# EUROPEAN PATENT APPLICATION

(11) **EP 4 266 536 A1**
(43) Date of publication of application: **25.10.2023**
(21) Application number: 22784810.8
(22) Date of filing: 21.03.2022
(51) Int. Cl.: H02J 7/00, A61B 5/00, A61B 5/024, A61B 5/021, A61B 5/01

(54) **CHARGING MOUNT APPARATUS FOR WEARABLE DEVICE**

(30) Priority: 07.04.2021 KR 20210045320
(71) Applicant: Samsung Electronics Co., Ltd., Suwon-si, Gyeonggi-do 16677 (KR)
(72) Inventor: NAM, Minhyuk, Suwon-si Gyeonggi-do 16677 (KR); KIM, Yongyi, Suwon-si Gyeonggi-do 16677 (KR); PARK, Sunghwa, Suwon-si Gyeonggi-do 16677 (KR); SAGONG, Jin, Suwon-si Gyeonggi-do 16677 (KR); LEE, Seungho, Suwon-si Gyeonggi-do 16677 (KR); CHANG, Juhee, Suwon-si Gyeonggi-do 16677 (KR); HAN, Kiwook, Suwon-si Gyeonggi-do 16677 (KR)
(74) Representative: Gulde & Partner
(86) International application number: PCT/KR2022/003914
(87) International publication number: WO 2022/215900

(57) **Abstract**

A charging mount apparatus for a wearable device according to one embodiment of the present invention may comprise: a mount housing comprising a first part, a second part, and a ring-shaped mounting space located between the first part and the second part and having the wearable device mounted therein; a fixed member fixed to the first part; a movable member comprising a wireless charging module, disposed on the first part, and moving to the ring-shaped mounting space from the fixed member; and at least one resilient body disposed between the fixed member and the movable member, and providing a force pushing the movable member to the ring-shaped mounting space. Various other embodiments may be possible.

## Description

### [Technical Field]

Various embodiments of the disclosure relate to a charging mount apparatus for a wearable device.

### [Background Art]

Among wearable devices worn on a user's human body, smart rings worn on the finger have been released in various sizes ranging from No. 5 to No. 10 indicating the size of the diameter thereof because each person's finger size is different.

Therefore, cradles for charging smart rings released in various diameter sizes are also manufactured according to the diameter sizes of the smart rings.

### [Disclosure of Invention]

### [Technical Problem]

However, in case that charging mount apparatuses are manufactured according to diameter sizes of smart rings, respectively, not only production costs are high but also inventory costs may increase since various types of components should be stocked for after-sales service (A/S).

### [Solution to Problem]

Various embodiments of the disclosure may provide a charging mount apparatus capable of mounting wearable devices such as smart rings having different inner diameter sizes, in one charging mount apparatus, even though the inner diameter sizes of smart rings are different.

In a charging mount apparatus according to various embodiments of the disclosure, a charging mount apparatus of a wearable device may include a cradling housing including a first portion, a second portion, and a ring-shaped cradling space which is positioned between the first portion and the second portion and in which the wearable device is mounted, a fixation member fixed to the first portion, a movable member which includes a wireless charging module, is disposed in the first portion, and is configured to move from the fixation member into the ring-shaped cradling space, and at least one elastic body disposed between the fixation member and the movable member so as to provide a force to push the movable member toward the ring-shaped cradling space.

A charging mount apparatus according to various embodiments of the disclosure may include a cradling housing including a ring-shaped cradling space in which the wearable device is mounted, a first guide member configured to move from the center of the ring-shaped cradling space to the ring-shaped cradling space, and a second guide member disposed to face the first guide member and configured to move from the center of the ring-shaped cradling space to the ring-shaped cradling space, a movable member disposed on the second guide member and configured to be movable to the ring-shaped cradling space, a first elastic body disposed between the second guide member and the movable member so as to provide a force to push the movable member into the ring-shaped cradling space, and a second elastic body disposed between the cradling housing and the first guide member so as to provide a force to push the first guide member toward the center of the cradling housing.

### [Advantageous Effects of Invention]

A charging mount apparatus according to various embodiments of the disclosure may mount and charge wearable devices such as smart rings having various diameter sizes.

### [Brief Description of Drawings]

FIG. 1 is a perspective view showing a wearable device according to various embodiments of the disclosure.
FIG. 2 is a cross-sectional view showing a wearable device cut along line A-A' in FIG. 1.
FIG. 3 is a plan view showing a charging mount apparatus according to various embodiments of the disclosure.
FIG. 4 a cross-sectional view showing a charging mount apparatus cut along line B-B' in FIG. 3.
FIG. 5 is a cross-sectional view showing a state before a wearable device is mounted in a charging mount apparatus according to various embodiments of the disclosure.
FIG. 6 is a plan view showing a charging mount apparatus according to various other embodiments of the disclosure.
FIG. 7 is a cross-sectional view showing a charging mount apparatus to which a cover is coupled, according to various other embodiments of the disclosure.
FIG. 8 is a cross-sectional view showing a cover according to various embodiments of the disclosure.

### [Best Mode for Carrying out the Invention]

Hereinafter, various embodiments of the disclosure will be described with reference to the accompanying drawings. However, it should be appreciated that they are not intended to limit the disclosure to particular embodiments, and the disclosure should be construed to cover various modifications, equivalents, and/or alternatives of the embodiments of the disclosure. With regard to the description of the drawings, the same or like reference signs may be used to designate the same or like elements.

An electronic device according to various embodiments of the disclosure may include at least one of, for example, a smart phone, a tablet personal computer (PC), a mobile phone, a video phone, an electronic book reader (e-book reader), a desktop PC, a laptop PC, a netbook computer, a workstation, a server, a personal digital assistant (PDA), a portable multimedia player (PMP), a MP3 player, a mobile medical device, a camera, and a wearable device (e.g., smart glasses, head-mounted device (HMD)), electronic clothing, electronic bracelet, electronic necklace, electronic accessory (appcessory), electronic tattoo, smart mirror, or smart watch).

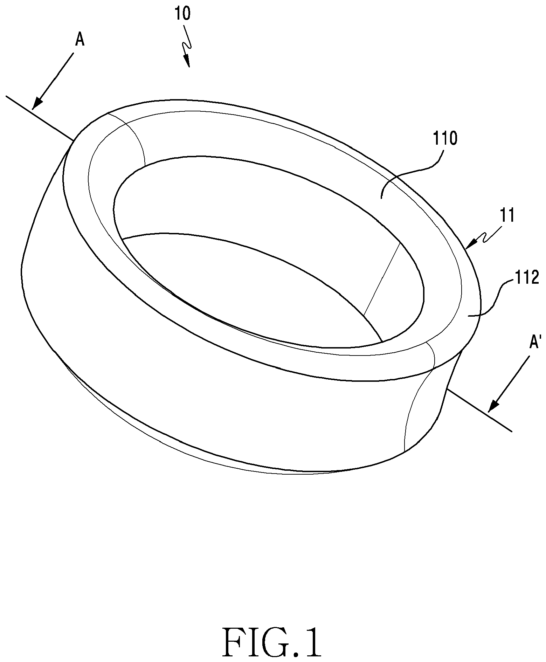

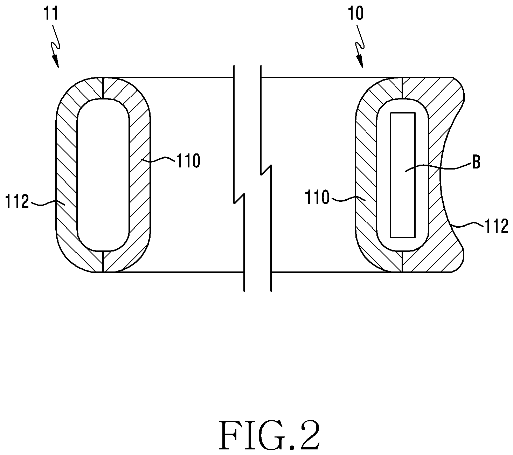

FIG. 1 is a perspective view showing a wearable device according to various embodiments of the disclosure. FIG. 2 is a cross-sectional view showing a wearable device cut along line A-A' in FIG. 1.

Referring to FIG. 1 and FIG. 2, an electronic device according to various embodiments may be a wearable device 10, and may be a ring-type wearable device 10 worn on a specific region, for example, a finger, of a human body. According to various embodiments, the wearable device 10 may include a ring-shaped housing 11.

According to various embodiments, the ring-shaped housing 11 of the wearable device 10 may include at least one biometric sensor (not shown), at least one printed circuit board (not shown), and at least one battery B. According to various embodiments, the wearable device 10 may have at least one biometric sensor disposed in the ring-shaped housing 11. For example, the biometric sensor may be used as a heart rate measurement device, a blood pressure measurement device, or a body temperature measurement device, and may detect a user's sleep state.

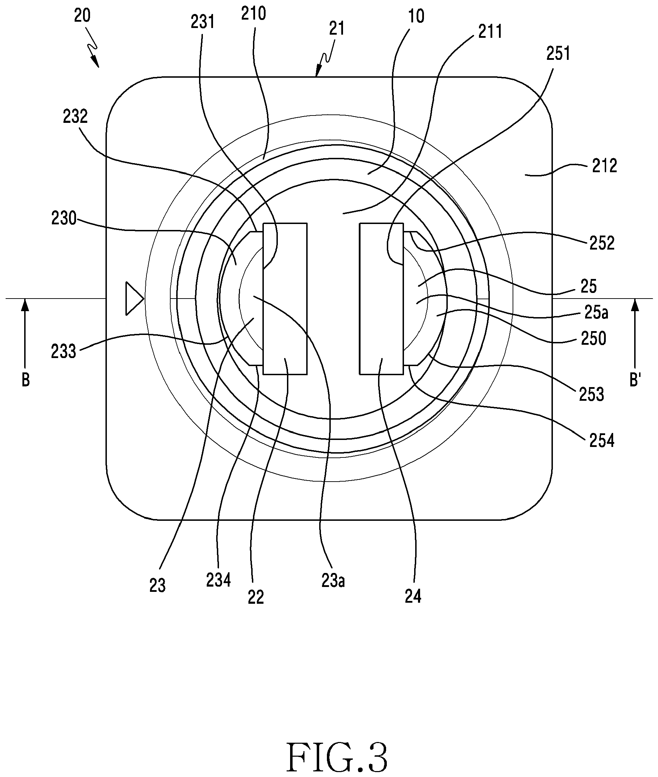

According to various embodiments, the ring-shaped housing 11 may include a first housing 110 and a second housing 112. For example, the ring-shaped housing 11 may be formed by coupling the first housing 110 and the second housing 112. According to various embodiments, at least a portion of the first housing 110 may be a portion in contact with a human body, and in case of being mounted in a charging mount apparatus (e.g., the charging mount apparatus 20 in FIG. 3), may be a portion in close contact with to the charging mount apparatus.

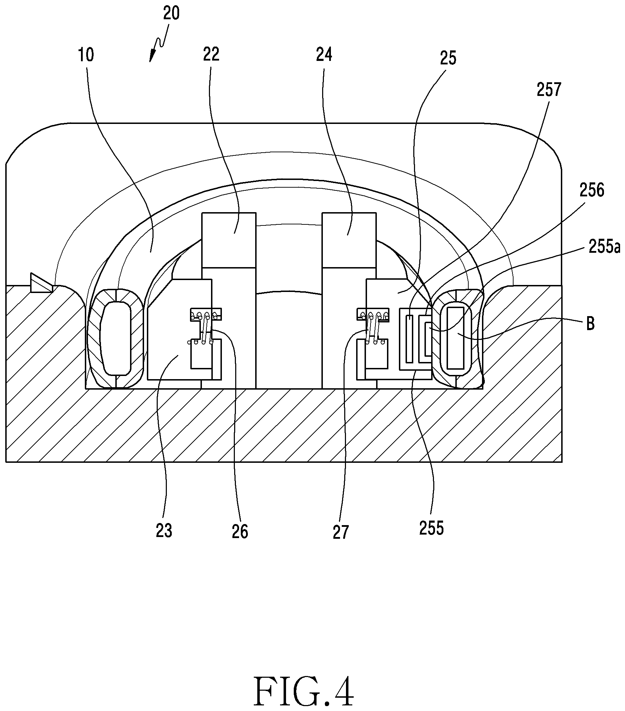

FIG. 3 is a plan view showing a charging mount apparatus according to various embodiments of the disclosure. FIG. 4 a cross-sectional view showing a charging mount apparatus cut along line B-B' illustrated in FIG. 3.

Referring to FIG. 3 and FIG. 4, according to various embodiments, a charging mount apparatus 20 for mounting and charging the wearable device 10 may include a cradling housing 21, at least one fixation member 22 and 24, at least one movable member 23 and 25, at least one and more elastic body 26 and 27, at least one charging interface (not shown), and/or a battery (not shown). For example, the at least one charging interface may include a USB port.

According to various embodiments, the cradling housing 21 may include a ring-shaped cradling space 210 for mounting the wearable device 10 therein. According to various embodiments, the cradling housing 21 may include a first portion 211, a second portion 212, and the ring-shaped cradling space 210 provided between the first portion 211 and the second portion 212. For example, the first portion 211 may be positioned in the center portion of the cradling housing 21 and include an inner portion positioned inside the ring-shaped cradling space 210, and the second portion 212 may be positioned in an outer portion outside the ring-shaped cradling space 210 of the cradling housing 21.

According to various embodiments, the ring-shaped cradling space 210 may be a space in which the wearable device 10 is mounted, and may have an upwardly open shape.

According to various embodiments, the at least one fixation member 22 and 24, the at least one movable member 23 and 25, and the at least one elastic body 26 and 27 may be arranged in the first portion 211, and the ring-shaped cradling space 210 may be positioned to surround the first portion 211. According to various embodiments, the fixation member 22 and 24 may be fixed to the first portion 211 of the cradling housing 21, and the movable member 23 and 25 may be arranged to be movable from the fixation member 22 and 24 to the ring-shaped cradling space 210. According to various embodiments, the fixation member may include a first fixation member 22 and a second fixation member 24, and the first fixation member 22 and the second fixation member 24 may face each other and be formed in an upright state on the first portion 211 of the cradling housing 21. According to various embodiments, the first fixation member 22 may be a support member for a first elastic body 26 providing a pushing force to the first movable member 23, and the second fixation member 24 may be a support member for a second elastic body 27 providing a pushing force to the second movable member 25.

According to various embodiments, the movable member may include a first movable member 23 and a second movable member 25 moving from the fixation members 22 and 24 to the ring-shaped cradling space 210, respectively. According to various embodiments, the first movable member 23 and the second movable member 25 may be arranged in the first portion 211 of the cradling housing 21, and may move in directions closer to or away from each other in the ring-shaped cradling space 210.

According to various embodiments, the first movable member 23 may be disposed on a surface among the outer surfaces of the first fixation member 22, which faces the ring-shaped cradling space 210, and the second movable member 25 may be disposed on a surface among the outer surface of the second fixation member 24, which faces the ring-shaped cradling space 210. According to various embodiments, the first elastic body 26 may be disposed between the first fixation member 22 and the first movable member 23, and the second elastic body 27 may be disposed between the second fixation member 24 and the second movable member 25. According to various embodiments, in case that the wearable device 10 is disposed in the ring-shaped cradling space 210 of the charging mount apparatus 20, the first elastic body 26 may provide a force forcing the first movable member 23 against the wearable device 10, and the second elastic body 27 may provide a force forcing the second movable member 25 against the wearable device 10.

According to various embodiments, the first elastic body 26 and/or the second elastic body 27 may be configured as a compression coil spring so that the first elastic body 26 provides a force to restrictively push the first movable member 23 toward the ring-shaped cradling space 210, and the second elastic body 27 provides a force to restrictively push the second movable member 23 toward the ring-shaped cradling space 210. According to various embodiments, the first movable member 23 may be coupled to the first fixation member 22 and thus may limitedly move from the first fixation member 22, and the second movable member 25 may be coupled to the second fixation member 24 and thus may limitedly move from the second fixation member 24. The coupling structure between the first fixation member 22 and the first movable member 23 and the coupling structure between the second fixation member 24 and the second movable member 25 are not illustrated in the drawings.

According to various embodiments, the first movable member 23 and the second movable member 25 may move in directions away from each other or in directions closer to each other. For example, the first movable member 23 and the second movable member 25 may move in directions away from each other in case that the wearable device 10 is not mounted, and may come into close contact with the wearable device 10 in case that the wearable device 10 is mounted.

According to various other embodiments, the first movable member 23 and/or the second movable member 25 may include at least one wireless charging module 255. According to various embodiments, the wireless charging module 255 may include a wireless charging antenna 256 and/or a charging circuit 257. According to various embodiments, the wireless charging module 255 may be included in either or both of the first movable member 23 and/or the second movable member 25.

According to various embodiments, the wireless charging antenna 256 may include a wireless charging coil 255a formed by patterning a conductive material in a coil shape (e.g., spiral shape) on a flexible circuit board (FPCB). According to various embodiments, in case that the wearable device 10 is mounted in the ring-shaped cradling space 210, the wearable device may come into proximity and face-to-face with the wireless charging coil 255a so that the battery B of the wearable device 10 is wirelessly charged. According to an embodiment, the wearable device 10 may include a wireless power reception coil (not shown) and a charging circuit (not shown) for charging the battery B by wirelessly receiving power from the charging mount apparatus 20. For example, in case that the wearable device 10 is positioned in the ring-shaped cradling space 210, the wireless power reception coil of the wearable device 10 may receive power from the wireless charging coil 255a, and thus the charging circuit of the wearable device 10 may charge the battery B, based on the received power.

According to various embodiments, the first movable member 23 may include an upper surface part 23a and multiple first surfaces 231 to 234, and at least a part of the first surfaces 231 to 234 and the upper surface part 23a may be cut such that a first inclined surface 230 is formed. According to various embodiments, the second movable member 25 may include an upper surface part 25a and multiple second surfaces 251-254, and at least a part of the second surfaces 251 to 254 and the upper surface part 25a may be cut such that a second inclined surface 250 is formed. According to various embodiments, in case that the wearable device 10 is mounted in the cradling housing 21, the first surface 233 and the second surface 253 positioned in the ring-shaped cradling space 210 may be proximity with the wearable device 10. For example, the first surface 233 and the second surface 253 positioned in the ring-shaped cradling space 210 may have a shape corresponding to the inner diameter surface of the first housing 110 to face the first housing 110 of the wearable device 10.

According to various embodiments, the wearable device 10 may be easily mounted in the ring-shaped cradle space 210 by the first inclined surface 230 and the second inclined surface 250. For example, in case of mounting the wearable device 10 therein, a part of the wearable device 10 may slide along the first inclined surface 230 and/or the second inclined surface 250 and thus may be smoothly mounted in the ring-shaped cradling space 210.

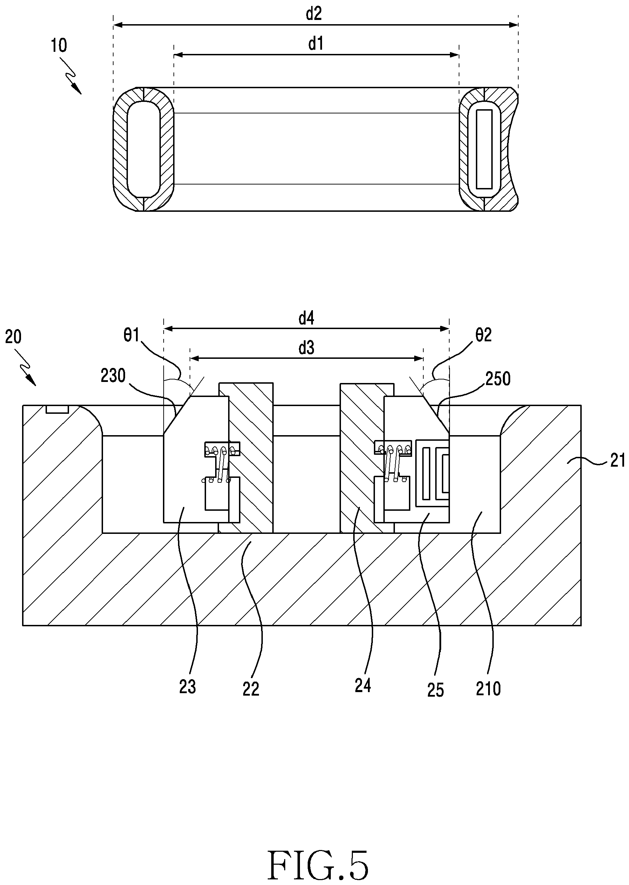

FIG. 5 is a cross-sectional view showing a state before a wearable device is mounted in a charging mount apparatus according to various embodiments of the disclosure.

Referring to FIG. 5, according to various embodiments, the wearable device 10 may have a size of an inner diameter d1 and a size of an outer diameter d2, and in consideration of the size of the inner diameter d1, the arrangement positions of the first movable member 23 and second movable member 25 and the inclination degrees of the first inclined surface 230 and the second inclined surface 250 may be determined.

According to various embodiments, a permissible range of the size of the inner diameter d1 of the wearable device 10 may be adjusted according to the inclination degrees of the first inclined surface 230 and the second inclined surface 250. According to various embodiments, in case that the inclination degrees (e.g., chamfer values) of the first inclined surface 230 and the second inclined surface 250 are large, the permissible range may be small, and in case that the inclination degrees are small, the permissible range of the size of the inner diameter may be large. For example, the permissible range of the size of the inner diameter d1 may be larger in case that the inclination angle O1 of the first inclined surface 230 and/or the inclination angle Θ2 of the second inclined surface 250 is 30 degrees than the case that the inclination angle of the first inclined surface and/or the inclination angle of the second inclined surface is 60 degrees. For example, the inclination angle O1 of the first inclined surface 230 and the inclination angle Θ2 of the second inclined surface 250 may be identical or different.

According to various embodiments, the size of the inner diameter d1 of the wearable device, which allows the wearable device to be mounted in the charging mount apparatus 30, may be adjusted between a first distance d3 and a second distance d4 which are distances between the first movable member 23 and the second movable member 24. According to various embodiments, the first distance d3 and the second distance d4 may be adjusted according to the inclination degrees of the first inclined surface 230 and the second inclined surface 250.

According to various embodiments, in case that the first movable member 23 and the second movable member 25 are maximally compressed toward the first fixation member 22 and the second fixation member 24, respectively, the fourth distance d4 may be smaller than the first distance d1, and in case that the first movable member 23 and the second movable member 25 are farthest from the first fixation member 22 and the second fixation member 24, respectively, the third distance d3 remain smaller than the first distance d1, and thus the wearable device may be easy mounted.

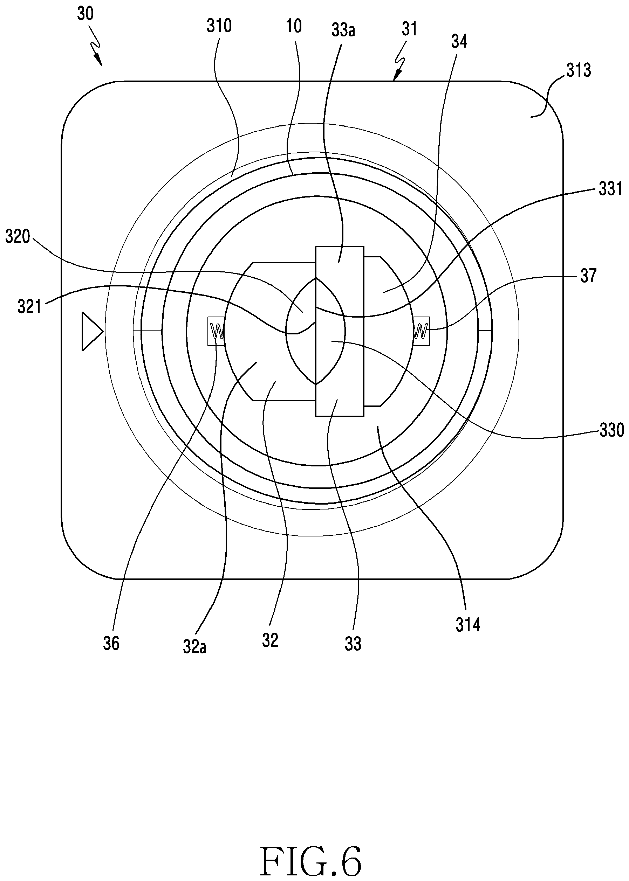

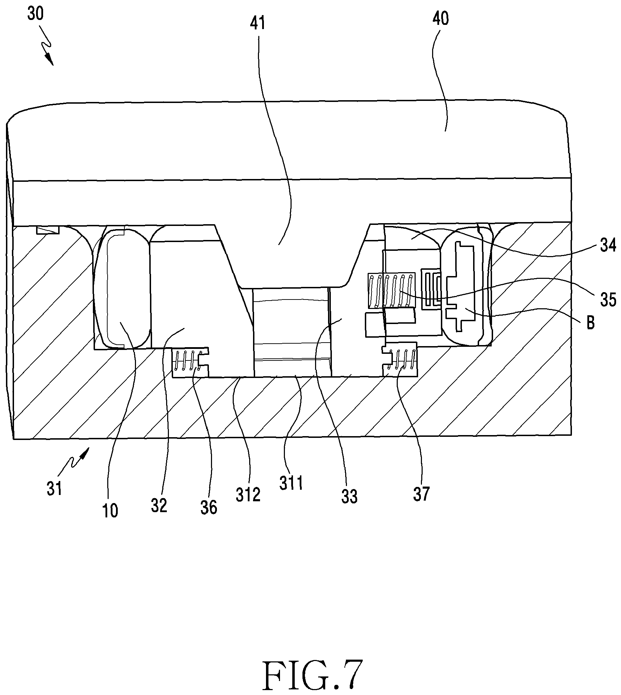

FIG. 6 is a plan view showing a charging mount apparatus according to various other embodiments of the disclosure. FIG. 7 is a cross-sectional view showing a charging mount apparatus to which a cover is coupled according to various other embodiments of the disclosure.

Referring to FIG. 6 and FIG. 7, according to various embodiments, a charging mount apparatus 30 may include a cradling housing 31. According to various embodiments, the cradling housing 31 may include a first portion 313, a second portion 314, and the ring-shaped cradling space 310. According to various embodiments, the ring-shaped cradling space 310 may be a space provided between the first portion 313 and the second portion 314, and may be a space in which the wearable device 10 can be mounted. According to various embodiments, the first portion 313 may be positioned in the center portion of the cradling housing 31 and include an inner portion positioned inside the ring-shaped cradling space 310, and the second portion 314 may be positioned in an outer portion outside the ring-shaped cradling space 310.

According to various embodiments, the cradling housing may include a first guide member 32, a second guide member 33, a movable member 34, and multiple elastic bodies 35, 36, and 37. According to various embodiments, the cradling housing 31 may include a ring-shaped cradling space 310 in which the wearable device 10 such as a smart ring is mounted. According to various embodiments, the ring-shaped cradling space 310 may be a space in which the ring-shaped wearable device 10 is mounted, and may be a space in which a part of the movable member 34 is movable to the ring-shaped cradling space 310.

According to various embodiments, the first guide member 32 and/or the second guide member 33 may be arranged to be movable by a guide rail 311 provided at the center of the cradling housing 31, be uprightly arranged, and be arranged to be movable in directions away from or closer to each other.

According to various embodiments, the guide rail 311 may be formed on the bottom portion 312 of the cradling housing 31.

According to various embodiments, the first guide member 32 may be disposed in the central area of the cradling housing 31, for example, in the central area of ring-shaped cradling space 310, and may be disposed to be movable to the ring-shaped cradling space 310 with reference to the center of the ring-shaped cradling space 310. According to various embodiments, the second guide member 33 may be disposed in the central area of the cradling housing 31, for example, in the central area of ring-shaped cradling space 310, and may be disposed to be movable to the ring-shaped cradling space 310 with reference to the center of the ring-shaped cradling space 310.

According to various embodiments, a first sliding groove 320 may be formed on an upper surface 32a and a first surface 321 of the first guide member 32, the first sliding groove being formed by cutting at least a part of a portion thereof facing the second guide member 33. According to various embodiments, a second sliding groove 330 may be formed on an upper surface 33a and a second surface 331 of the second guide member 33, the second sliding groove being formed by cutting at least a part of a portion thereof facing the first guide member 32. For example, a part of each of the first sliding groove 320 and the second sliding groove 330 may be a curved surface.

According to various embodiments, the second guide member 33 may include the movable member 34. According to various embodiments, the movable member 34 may move from the second guide member 33 to the ring-shaped cradling space 310. According to various embodiments, the movable member 34 may be coupled to the second guide member 33, and thus may move limitedly. For example, the movable member 34 may move away from the second guide member 33 within a limited distance.

According to various embodiments, the cradling housing 31 may include a first elastic body 35 to a third elastic body 37. For example, the first elastic body 35 to the third elastic body 37 may be compression coil springs providing a compression force. According to various embodiments, the first elastic body 35 may be disposed between the second guide member 33 and the movable member 34 so as to provide a force to push the movable member 34 toward the ring-shaped cradling space 310. According to various embodiments, the second elastic body 36 may be disposed between the cradling housing 31 and the first guide member 32 so as to provide a force to push the first guide member 32 toward the center of the cradling housing 31. According to various embodiments, the third elastic body 37 may be disposed between the cradling housing 31 and the second guide member 33 so as to provide a force to push the second guide member 33 toward the center of the cradling housing 31.

According to various embodiments, the first guide member 32 and the movable member 34 may be in close contact with the inner diameter surface (e.g., the inner diameter surface of the first housing 110) of the wearable device 10, and may provide a force to push the wearable device 10 to the ring-shaped cradling space 310.

According to various embodiments, the second elastic body 36 and the third elastic body 37 may be arranged along a direction in which the ring-shaped cradling space is oriented, and may face each other with reference to the center of the cradling housing 31.

According to various embodiments, in case that a cover 40 is not coupled to the cradling housing 31, the first guide member 32 and the second guide member 33 may be in close contact with each other, based on the pushing force of the second elastic body 36 and the third elastic body 37, and the first sliding groove 320 and the second sliding groove 330 may face each other. According to various embodiments, in case that the cover 40 is coupled to the cradling housing 31, the first guide member 32 and the second guide member 33 may move in directions away from each other by a sliding protrusion 41 of the cover 40, and the movable member 34 may move toward the cradling space 310. According to various embodiments, in case that the cradling housing 31 is closed by the cover 40 in a state where the wearable device 10 is mounted in the ring-shaped cradling space 310, by sliding between the sliding protrusion 41 and the first sliding groove 320 and/or the second sliding groove 330, the first guide member 32 and/or the second guide member 33 may move away from each other, and the movable member 34 may come into close contact with the mounted wearable device 10 and thus the close contact state thereof may be maintained.

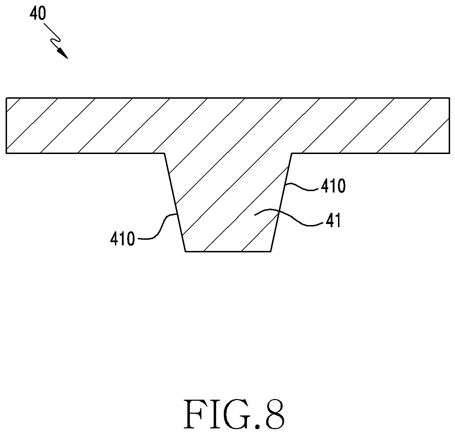

FIG. 8 is a cross-sectional view showing a cover according to various embodiments of the disclosure.

Referring to FIG. 8, a cradling housing (e.g., the cradling housing 31 illustrated in FIG. 7) according to various embodiments may be opened or closed by the cover 40. According to various embodiments, in case that the wearable device 10 is mounted in the cradling housing 31, when the cradling housing 31 is closed by the cover 40, the charging mount apparatus may be configured to include a structure forcing a movable member (e.g., the movable member 34 illustrated in FIG. 7) having a wireless charging module against the mounted wearable device 10. According to various embodiments, the cover 40 may include the sliding protrusion 41, and the sliding protrusion 41 may be formed in a shape protruding in one direction (e.g., in the lower direction) from one surface of the cover 40.

According to various embodiments, the sliding protrusion 41 may have a truncated conical shape, and the outer surface 410 thereof may be a sliding surface. However, the shape of the sliding protrusion 41 is not limit to the truncated cone shape.

According to various embodiments, in case that the cover 40 moves in a direction perpendicular to the cradling housing 31, that is, in case that the cradling housing is closed, the sliding protrusion 41 may slide with a first sliding groove and a second sliding groove (e.g., the first sliding groove 320 and the second sliding groove 330 illustrated in FIG. 7), continuously, the first guide member 32 and the second guide member 33 may move in directions away from each other, and a movable member (e.g., the movable member 34 illustrated in FIG. 7) may come into close contact with the mounted wearable device 10.

According to various embodiments, a charging mount apparatus (e.g., the charging mount apparatus 20 illustrated in FIG. 3) of a wearable device (e.g., the wearable device 10 illustrated in FIG. 1) may include a cradling housing (e.g., the cradling housing 21 illustrated in FIG. 3) including a first portion (e.g., the first portion 211 illustrated in FIG. 3), a second portion (e.g., the second portion 212 illustrated in FIG. 3), and a ring-shaped cradling space (e.g., the ring-shaped cradling space 210 illustrated in FIG. 3) which is positioned between the first portion (e.g., the first portion 211 illustrated in FIG. 3) and the second portion (e.g., the second portion 212 illustrated in FIG. 3) and in which the wearable device (e.g., the wearable device 10 illustrated in FIG. 1) is mounted, a fixation member (e.g., the fixation member 22 or 24 illustrated in FIG. 3) fixed to the first portion (e.g., the first portion 211 illustrated in FIG. 3), a movable member (e.g., a movable member 22 or 25 illustrated in FIG. 4) which includes a wireless charging module (e.g., the wireless charging module 255 illustrated in FIG. 4), is disposed in the first portion (e.g., the first portion 211 illustrated in FIG. 3), and is configured to move from the fixation member (e.g., the fixation member 22 or 24 illustrated in FIG. 3) into the ring-shaped cradling space (e.g., the ring-shaped cradling space 210 illustrated in FIG. 3), and at least one elastic body (e.g., the elastic body 26 or 27 illustrated in FIG. 4) disposed between the fixation member (e.g., the fixation member 22 or 24 illustrated in FIG. 3) and the movable member (e.g., the movable member 22 or 25 illustrated in FIG. 4) so as to provide a force to push the movable member (e.g., the movable member 22 or 25 illustrated in FIG. 4) toward the ring-shaped cradling space (e.g., the ring-shaped cradling space 210 illustrated in FIG. 3).

According to various embodiments, the first portion (e.g., the first portion 211 illustrated in FIG. 3) may be the center portion of the cradling housing (e.g., the cradling housing 21 illustrated in FIG. 3), and the second portion (e.g., the second portion 212 illustrated in FIG. 3) may be an outer portion of the ring-shaped cradling space (e.g., the ring-shaped cradling space 210 illustrated in FIG. 3).

According to various embodiments, the fixation member (e.g., the fixation member 22 or 24 illustrated in FIG. 3) may include a first fixation member (e.g., the fixation member 22 illustrated in FIG. 3) and a second fixation member (e.g., the fixation member 24 illustrated in FIG. 3) facing each other, and the movable member (e.g., the movable member 23 or 25 illustrated in FIG. 4) may include a first movable member (e.g., the movable member 23 illustrated in FIG. 4) configured to move from the first fixation member (e.g., the fixation member 22 illustrated in FIG. 3) into the ring-shaped cradling space (e.g., the ring-shaped cradling space 210 illustrated in FIG. 3), and a second movable member (e.g., the movable member 25 illustrated in FIG. 4) configured to move from the second fixation member (e.g., the fixation member 24 illustrated in FIG. 3) into the ring-shaped cradling space (e.g., the ring-shaped cradling space 210 illustrated in FIG. 3).

According to various embodiments, the elastic body (e.g., the elastic body 26 or 27 illustrated in FIG. 4) may include a first elastic body (e.g., the elastic body 26 illustrated in FIG. 4) disposed between the first fixation member (e.g., the fixation member 22 illustrated in FIG. 3) and the first movable member so as to provide a force forcing the first movable member (e.g., the movable member 23 illustrated in FIG. 4) against the wearable device (e.g., the wearable device 10 illustrated in FIG. 1), and a second elastic body (e.g., the elastic body 27 illustrated in FIG. 4) disposed between the second fixation member (e.g., the fixation member 24 illustrated in FIG. 3) and the second movable member (e.g., the movable member 25 illustrated in FIG. 4) so as to provide a force forcing the second movable member (e.g., the movable member 25 illustrated in FIG. 4) against the wearable device (e.g., the wearable device 10 illustrated in FIG. 1).

According to various embodiments, the first elastic body (e.g., the elastic body 26 illustrated in FIG. 4) and the second elastic body (e.g., the elastic body 27 illustrated in FIG. 4) may be arranged along a direction toward the ring-shaped cradling space (e.g., the ring-shaped cradling space 210 illustrated in FIG. 3).

According to various embodiments, the wireless charging module (e.g., the wireless charging module 255 illustrated in FIG. 4) may be disposed in either or both of the first movable member (e.g., the movable member 23 illustrated in FIG. 4) and the second movable member (e.g., the movable member 25 illustrated in FIG. 4).

According to various embodiments, the first movable member (e.g., the movable member 23 illustrated in FIG. 4) and the second movable member (e.g., the movable member 25 illustrated in FIG. 4) may have inclined surfaces (e.g., the inclined surfaces 230 and 250 illustrated in FIG. 3) formed by cutting at least a part of each of the upper surface parts thereof.

According to various embodiments, the wearable device (e.g., the wearable device 10 illustrated in FIG. 1) may include a ring-shaped housing (e.g., the ring-shaped housing 11 illustrated in FIG. 1), and at least one battery (e.g., the battery B illustrated in FIG. 2) may be disposed along the ring-shaped housing (e.g., the ring-shaped housing 11 illustrated in FIG. 1).

According to various embodiments, the movable member (e.g., the movable member 23 or 25 illustrated in FIG. 4) may be configured to be in close contact with the inner diameter surface of the ring-shaped housing (e.g., the ring-shaped housing 11 illustrated in FIG. 1) and provide a force to push the ring-shaped housing (e.g., the ring-shaped housing 11 illustrated in FIG. 1) into the ring-shaped cradling space (e.g., the ring-shaped cradling space 210 illustrated in FIG. 3).

According to various embodiments, the wearable device (e.g., the wearable device 10 illustrated in FIG. 1) may include at least one biometric sensor, and the at least one biometric sensor may be configured to be operable as a heart rate measurement device, a blood pressure measurement device, or a body temperature measurement device.

According to various embodiments, a charging mount apparatus (e.g., the charging mount apparatus 20 illustrated in FIG. 3) of a wearable device (e.g., the wearable device 10 illustrated in FIG. 1) may include a cradling housing (e.g., the cradling housing 21 illustrated in FIG. 3) including a ring-shaped cradling space (e.g., the ring-shaped cradling space 210 illustrated in FIG. 3) in which the wearable device (e.g., the wearable device 10 illustrated in FIG. 1) is mounted, a first guide member (e.g., the guide member 32 illustrated in FIG. 6) configured to move from the center of the ring-shaped cradling space (e.g., the ring-shaped cradling space 210 illustrated in FIG. 3) to the ring-shaped cradling space (e.g., the ring-shaped cradling space 210 illustrated in FIG. 3), and a second guide member (e.g., the guide member 33 illustrated in FIG. 6) disposed to face the first guide member (e.g., the guide member 32 illustrated in FIG. 6) and configured to move from the center of the ring-shaped cradling space (e.g., the ring-shaped cradling space 210 illustrated in FIG. 3) to the ring-shaped cradling space (e.g., the ring-shaped cradling space 210 illustrated in FIG. 3), a movable member (e.g., the movable member 32 or 34 illustrated in FIG. 6) disposed on the second guide member (e.g., the guide member 33 illustrated in FIG. 6) and configured to be movable to the ring-shaped cradling space (e.g., the ring-shaped cradling space 210 illustrated in FIG. 3), a first elastic body (e.g., the first elastic body 35 illustrated in FIG. 6) disposed between the second guide member (e.g., the guide member 33 illustrated in FIG. 6) and the movable member (e.g., the movable member 32 or 34 illustrated in FIG. 6) so as to provide a force to push the movable member (e.g., the movable member 32 or 34 illustrated in FIG. 6) into the ring-shaped cradling space (e.g., the ring-shaped cradling space 210 illustrated in FIG. 3), and a second elastic body (e.g., the second elastic body 36 illustrated in FIG. 6) disposed between the cradling housing (e.g., the cradling housing 21 illustrated in FIG. 3) and the first guide member (e.g., the guide member 32 illustrated in FIG. 6) so as to provide a force to push the first guide member (e.g., the guide member 32 illustrated in FIG. 6) toward the center of the cradling housing (e.g., the cradling housing 21 illustrated in FIG. 3).

According to various embodiments, a third elastic body (e.g., the third elastic body 37 illustrated in FIG. 6) disposed in the cradling housing (e.g., the cradling housing 21 illustrated in FIG. 3) and the second guide member (e.g., the guide member 33 illustrated in FIG. 6) so as to provide a force to push the second guide member (e.g., the guide member 33 illustrated in FIG. 6) toward the center of the housing may be included therein.

According to various embodiments, the third elastic body (e.g., the third elastic body 37 illustrated in FIG. 6) may be disposed to face the second elastic body (e.g., the second elastic body 36 illustrated in FIG. 6) with reference to the center of the ring-shaped cradling space (e.g., the ring-shaped cradling space 210 illustrated in FIG. 3).

According to various embodiments, the first guide member (e.g., illustrated guide member 32 in FIG. 6) may have a first sliding groove (e.g., the sliding groove 320 illustrated in FIG. 6) formed on the upper surface thereof, and the second guide member (e.g., the guide member 33 illustrated in FIG. 6) may have a second sliding groove (e.g., the sliding groove 330 illustrated in FIG. 6) formed on the upper surface thereof.

According to various embodiments, in a state where the wearable device (e.g., the wearable device 10 illustrated in FIG. 1) is not mounted in the cradling housing (e.g., the cradling housing 21 illustrated in FIG. 3), the first sliding groove (e.g., the sliding groove 320 illustrated in FIG. 6) and the second sliding groove (e.g., the sliding groove 330 illustrated in FIG. 6) may face each other.

According to various embodiments, a cover configured to open or close the cradling housing (e.g., the cradling housing 21 illustrated in FIG. 3) may be included therein, and the cover may include a sliding protrusion (e.g., the sliding protrusion 41 illustrated in FIG. 8) provided at the center thereof and configured to slide with the first sliding groove (e.g., the sliding groove 320 illustrated in FIG. 6) and the second sliding groove (e.g., the sliding groove 330 illustrated in FIG. 6).

According to various embodiments, the sliding protrusion (e.g., the sliding protrusion 41 illustrated in FIG. 8) may have a truncated cone shape.

According to various embodiments, in case that the cradling housing (e.g., the cradling housing 21 illustrated in FIG. 3) is closed by the cover (e.g., the cover 40 illustrated in FIG. 8), the first guide member (e.g., the guide member 32 illustrated in FIG. 6) and the second guide member (e.g., the guide member 33 illustrated in FIG. 6) may be configured to move in directions away from each other by sliding between the sliding protrusion and the first sliding groove (e.g., the sliding groove 320 illustrated in FIG. 6) and the second sliding groove (e.g., the sliding groove 330 illustrated in FIG. 6).

According to various embodiments, a guide rail (e.g., guide rail 311 illustrated in FIG. 7), along which the first guide member (e.g., the guide member 32 illustrated in FIG. 6) and the second guide member (e.g., the guide member 33 in FIG. 6) move, may be formed on the bottom portion of the cradling housing (e.g., the cradling housing 21 illustrated in FIG. 3).

According to various embodiments, the first guide member (e.g., the guide member 32 in FIG. 6) and the movable member (e.g., the movable member 32 or 34 in FIG. 6) may be configured to be in close contact with the inner diameter surface of the wearable device (e.g., the wearable device 10 illustrated in FIG. 1) and provide a force to push the wearable device (e.g., the wearable device 10 illustrated in FIG. 1) into the ring-shaped cradling space (e.g., the ring-shaped cradling space 210 illustrated in FIG. 3).

Various embodiments of the disclosure described and shown in the specification and the drawings are merely specific examples that have been presented to easily explain the technical contents of the disclosure and help understanding of the disclosure, and are not intended to limit the scope of the disclosure. Therefore, the scope of the disclosure should be construed to include, in addition to the embodiments disclosed herein, all changes and modifications derived on the basis of the technical idea of the disclosure.

## Claims

1. A charging mount apparatus of a wearable device, comprising:
a cradling housing comprising a first portion, a second portion, and a ring-shaped cradling space which is positioned between the first portion and the second portion and in which the wearable device is mounted;
a fixation member fixed to the first portion;
a movable member which comprises a wireless charging module, is disposed in the first portion, and is configured to move from the fixation member into the ring-shaped cradling space; and
at least one elastic body disposed between the fixation member and the movable member so as to provide a force to push the movable member toward the ring-shaped cradling space.

2. The charging mount apparatus of claim 1, wherein the first portion is the center portion of the cradling housing, and the second portion is an outer portion of the ring-shaped cradling space.

3. The charging mount apparatus of claim 2, wherein the fixation member comprises a first fixation member and a second fixation member facing each other,
and wherein the movable member comprises:
a first movable member configured to move from the first fixation member into the ring-shaped cradling space; and
a second movable member configured to move from the second fixation member into the ring-shaped cradling space.

4. The charging mount apparatus of claim 3, wherein the elastic body comprises:
a first elastic body disposed between the first fixation member and the first movable member so as to provide a force forcing the first movable member against the wearable device; and
a second elastic body disposed between the second fixation member and the second movable member so as to provide a force forcing the second movable member against the wearable device.

5. The charging mount apparatus of claim 4, wherein the first elastic body and the second elastic body are arranged along a direction toward the ring-shaped cradling space.

6. The charging mount apparatus of claim 4, wherein the wireless charging module is disposed in either or both of the first movable member and the second movable member.

7. The charging mount apparatus of claim 3, wherein the first movable member and the second movable member have inclined surfaces formed by cutting at least a part of each of the upper surface parts thereof.

8. The charging mount apparatus of claim 1, wherein the wearable device comprises a ring-shaped housing, and at least one battery is disposed along the ring-shaped housing.

9. The charging mount apparatus of claim 8, wherein the movable member is configured to be in close contact with the inner diameter surface of the ring-shaped housing and provide a force to push the ring-shaped housing into the ring-shaped cradling space.

10. The charging mount apparatus of claim 1, wherein the wearable device comprises at least one biometric sensor, and the at least one biometric sensor is configured to be operable as a heart rate measurement device, a blood pressure measurement device, or a body temperature measurement device.

11. A charging mount apparatus of a wearable device, comprising:
a cradling housing comprising a ring-shaped cradling space in which the wearable device is mounted, a first guide member configured to move from the center of the ring-shaped cradling space to the ring-shaped cradling space, and a second guide member disposed to face the first guide member and configured to move from the center of the ring-shaped cradling space to the ring-shaped cradling space;
a movable member disposed on the second guide member and configured to be movable to the ring-shaped cradling space;
a first elastic body disposed between the second guide member and the movable member so as to provide a force to push the movable member into the ring-shaped cradling space; and
a second elastic body disposed between the cradling housing and the first guide member so as to provide a force to push the first guide member toward the center of the cradling housing.

12. The charging mount apparatus of claim 11, further comprising a third elastic body disposed in the cradling housing and the second guide member so as to provide a force to push the second guide member toward the center of the housing,
wherein the third elastic body is disposed to face the second elastic body with reference to the center of the ring-shaped cradling space.

13. The charging mount apparatus of claim 11, wherein the first guide member has a first sliding groove formed on the upper surface thereof and the second guide member has a second sliding groove formed on the upper surface thereof, and
wherein, in a state where the wearable device is not mounted in the cradling housing, the first sliding groove and the second sliding groove face each other.

14. The charging mount apparatus of claim 13, further comprising a cover configured to open or close the cradling housing,
wherein the cover comprises a sliding protrusion provided at the center thereof and configured to slide with the first sliding groove and the second sliding groove,
wherein the sliding protrusion has a truncated cone shape, and
wherein, in case that the cradling housing is closed by the cover, the first guide member and the second guide member are configured to move in directions away from each other by sliding between the sliding protrusion and the first sliding groove and the second sliding groove.

15. The charging mount apparatus of claim 11, wherein the first guide member and the movable member are configured to be in close contact with the inner diameter surface of the wearable device and provide a force to push the wearable device into the ring-shaped cradling space.
